# EUROPEAN PATENT APPLICATION

(11) **EP 1 566 621 A1**
(43) Date of publication of application: **24.08.2005**
(21) Application number: 02808162.8
(22) Date of filing: 21.11.2002
(51) Int. Cl.: G01N 1/10, G01N 33/487, A61B 10/00

(54) **INSTRUMENT FOR COLLECTING AND RECOVERING SALIVA**

(71) Applicant: Sapporo Immuno Diagnostic Laboratory, Hokkaido 001-0922 (JP)
(72) Inventor: YOKOYAMA, Toru, Sapporo Immuno Diagnostic Lab., Sapporo-shi, Hokkaido 001-0922 (JP); SHINOZUKA, Naoki, Sapporo Immuno Diagnostic Lab., Sapporo-shi, Hokkaido 001-0922 (JP)
(74) Representative: HOFFMANN EITLE
(86) International application number: PCT/JP2002/012167
(87) International publication number: WO 2004/046693

(57) **Abstract**

The present invention is **characterized by** a method, which simultaneously performs sterilization of the oral cavity, promotion of saliva secretion, and microsampling, at the time of saliva sampling intended for saliva tests including determination of glucose in saliva, and further provides a syringe type instrument for sampling and recovery of saliva with the use of inexpensive members for removing a salivary viscous component and recovering a salivary serous component, and a method for sampling saliva and recovering a salivary serous component with the use of the instrument.

## Description

### FIELD OF THE INVENTION.

This invention relates to a disposable simple instrument, which enables saliva to be collected and recovered for use in a saliva test using, as a sample, saliva from the salivary glands such as parotid gland, submandibular gland, and sublingual gland. The invention also relates to a method for sampling and recovering saliva using the disposable simple instrument.

### BACKGROUND OF THE INVENTION

The inventors have so far devised a method for sampling, and a film type instrument for sampling and recovering, oral liquid secreta for determination of salivary glucose (e.g. WO02/086453(2002)). They have also elucidated their bactericidal effect on oral bacteria which suppresses the degradation of glucose by bacteria (WO02/31105(2002)). Through these efforts, they have clarified a simple method for sampling and recovering saliva, and further the usefulness of saliva as a diagnostic material.

Currently, saliva tests (Annals of the New York Academy of Sciences, Vol. 694, p.216(1993)) are conducted with the use of a saliva sampling instrument "OraSure" (Epitope, Inc.) (e.g., WO97/24979(1997), WO 96/04850(1996), WO95/27205(1995)), a saliva sampling/testing instrument "OraQuick" (Epitope, Inc.) (e.g., WO99/50656(1999)), a saliva sampling instrument "Omnisal(Saliva·Sampler)" (Saliva Diagnostic Systems, Inc.) (e.g., WO95/02996(1995), U.S. Patent No. 5,260,031(1993)), a saliva sampling instrument "Salivette" (Sarstedt) (e.g., U.S. Patent No. 4,774,962(1988)), and a saliva sampling instrument "ORALscreen collector" (Avitar, Inc.) (e.g., U.S. Patent No. 6,200,275(2001)). These instruments are used in various screening tests for HIV, hepatitis virus, syphilis, rubella and measles, and drug monitoring tests on therapeutics such as theophylline and phenytoin, and pharmaceuticals such as nicotine and cocaine (The Japan Society for Clinical Laboratory Automation, Vol. 19, p.265(1994)).

In all of the above-mentioned saliva sampling instruments, the volume of the saliva absorption pad is several hundred cubic millimeters or more, and the saliva sampling time is as long as several minutes. In sampling saliva, an operation for promoting the secretion of saliva is not performed. To prevent the proliferation of oral bacteria present in the sample after saliva sampling, some of the instruments are impregnated with a preservative solution containing sodium azide (saliva sampling instrument "Omnisal(Saliva-Sampler)"). However, none of these instruments are subjected to sterilization before and during sampling. Nor is the determination of glucose included in the test items.

Next, in connection with the salivary glucose test of Yamaguchi et al., there have been descriptions of methods and examples of spraying and removing a cleaning solution before saliva sampling for the purpose of cleaning of the oral cavity with the use of a cleaning solution and removal of the cleaning solution (for example, Japanese Patent Application Laid-Open No. 1997-72900(1997)). However, neither a method for promoting saliva secretion nor a method for sterilizing the oral cavity is described. Furthermore, a saliva sampling portion-integrated sensor or colorimetric plate (for example, Japanese Patent Application Laid-Open No. 2000-9728(2000)) is quoted. As a means of promoting saliva secretion, there is a description of impartment of odor to the cleaning solution, or coating of the saliva sampling portion with a stimulant solution. Mention of variously shaped sampling instruments and cleaning of the salivary gland is also made. However, no description is presented of sterilization.

Various mouthwashes containing germicides are developed and sold as dentifrices (for example, WO91/18585(1991)). However, they are intended for preventing dental caries, and their taste substances and flavors are not aimed at promoting saliva secretion.

In recent years, saliva test as non-invasive monitoring are expanding and various POCT (point-of-care testing) instruments, for example biosensor, have been developed. For methods of taking and recovering samples, there is a strong demand for simple instruments which enable recovery immediately after convenient and prompt sampling.

As described above, no proposal has been made for a way of applying sterilization to oral bacteria when sampling saliva, at least when determining glucose in the saliva. There have been no proposal for promoting the secretion of saliva, and at the same time, sterilizing oral bacteria when sampling saliva, and for compact simple instruments which can perform recovery after sampling. Particularly, there have been no proposals for simple instruments which can achieve microsampling in a very short time for POCT use, and can immediately recover samples while specifically degrading assay inhibitors intrinsic to saliva.

The present invention provides a method and an instrument of a novel shape which enable microsampling, prompt recovery, and degradation of inhibitors to be performed in a shorter time than the film type sampling/recovery instrument (for example, WO02/086453(2002)) provided before by the present inventors.

### SUMMARY OF THE INVENTION

In an attempt to solve the above-described problems, the present invention provides a disposable, compact, simple instrument, which sterilizes bacteria present in the oral cavity, samples a trace amount of saliva promptly while promoting the secretion of saliva, and removes a viscous component, while degrading an intrinsic inhibitor in saliva, to achieve recovery of serous saliva, for use in saliva tests including determination of glucose in the saliva. The invention also provides a method for recovery of serous saliva by use of the instrument.

### DESCRIPTION OF THE INVENTION

The present invention is characterized first by performing intraoral bactericidal cleaning, and intraoral bactericidal dewatering of bacteria, which are present in wide varieties and in large amounts in the oral cavity and utilize glucose as a nutrition source, in determining glucose in saliva, thereby sterilizing the oral bacteria. For this procedure, pretreatment is performed using both of, or only the latter of, a mouthwash containing a bactericidal component and a saliva secretion promoting component, and a sublingual surplus water absorber comprising a non-woven fabric containing a bactericidal component and a saliva secretion promoting component.

Secondly, the present invention is characterized by using a sweetener, a sour agent, and a flavor for stimulating a gustatory sensation and an olfactory sensation as means for promoting saliva secretion, and performing sterilization and promotion of saliva secretion simultaneously.

Thirdly, the present invention is characterized by providing a downsized saliva absorber, which shortens the saliva sampling time for actualizing a prompt operation, concretely, a microsampling instrument containing a bactericidal component and a saliva secretion promoting component, and a syringe type simple instrument using inexpensive members capable of performing degradation of an inhibitor and recovery of a salivary serous component by a convenient operation, thereby achieving, with extreme rapidity and convenience, a process ranging from sampling involving sterilization and promotion of saliva secretion, and recovery involving degradation of an inhibitor and removal of a viscous component.

The inventors have also found the bactericidal effect of a quaternary ammonium salt-based bactericidal component on the Streptococcus mutans group, etc. which are dental caries-causing bacteria present in the oral cavity, from changes in glucose over time (for example, WO02/31105(2002)). Thus, the present invention combines an antibacterial component with the above-mentioned bactericidal component, and provides a pretreatment instrument and a saliva sampling instrument which add an antibacterial function, which suppresses the proliferation of residual organisms, to a bactericidal function.

The present invention also provides a mouthwash and an instrument for pretreatment, and a saliva sampling instrument, which contain, in addition to the above-mentioned bactericidal component, a suitable sweetening component, a suitable sour component, and a suitable flavor component not impeding saliva tests including glucose determination, with the aim of promoting saliva secretion.

The instrument for sampling and recovery of saliva according to the present invention comprises a swab type sampling instrument and a syringe type recovery instrument. The swab type sampling instrument corresponding to a piston has, at one end, a conical saliva absorber for microsampling, has a gasket disposed at the bottom of a cone, and has, at its opposite and, a pressing pad used for insertion into the syringe type recovery instrument and for compression.

The syringe type recovery instrument has, at one end, a wide opening portion for facilitating the insertion of the saliva absorber and also facilitating the engagement of fingers during a pressing operation, and has, at its opposite end, a saliva absorber compressing portion, at the tip of which is, a recovery hole for recovering a salivary serous component. On the inner surface of the recovery hole, a trace amount piece of the same constituent as that of the saliva absorber is disposed for use as a filter layer.

As a method for degrading a saliva-intrinsic inhibitor serving as an impediment to a saliva test, a suitable enzyme or reagent component for degradation is immobilized to or incorporated into the inner wall surface and/or the filter layer of the syringe type recovery instrument which is not directly used in the oral cavity. By this measure, degradation is completed when the salivary serous component is recovered.

The saliva absorber and the compressing portion of the syringe type recovery instrument are formed in a conical shape. As a result, a dead space produced during pressing can be minimized as compared with other shapes such as a cylindrical shape, and waste-free recovery can be achieved.

To achieve further and prompt microsampling, the volume of the saliva absorber should desirably be set at 150 mm³ or less.

In an other syringe type saliva sampling instrument (for example, U.S. Patent No. 5,830,410(1998)), the saliva absorbing pad has a volume of about 3,000 mm³, and is not used for microsampling. Moreover, this pad contains 3.5 wt.% sodium chloride for antibacterial purposes and 0.3 wt.% citric acid for promotion of saliva secretion, but has not attained the bactericidal effect and promotion of saliva secretion that we have sought. The test items of conventional methods do not include determination of glucose.

As described above, the syringe type simple instrument of the present invention, containing a bactericidal component and a saliva secretion promoting component and arranged to be used for sampling and recovering saliva, has achieved convenient operability of the process from sampling until recovery of a salivary serous component which has degraded an inhibitor.

### BRIEF DESCRIPTION OF THE DRAWINGS

FIGS. 1(a) to 1(c) are views showing a saliva sampling instrument and a salivary serous component recovery instrument in Example 1 of the present invention, in which
FIG. 1(a) is a longitudinal sectional view of a saliva sampling instrument,
FIG. 1(b) is a longitudinal sectional view of a salivary serous component recovery instrument, and
FIG. 1(c) is a longitudinal sectional view of the salivary serous component recovery instrument into which the saliva sampling instrument has been inserted, and
FIG. 2 is a schematic view showing a method for sampling saliva and recovering a salivary serous component.

The numerals in the drawings are described as follows:

1 is a saliva sampling instrument; 2, a saliva absorber; 3, a piston rod; 4, a gasket; 5, a pressing pad; 6, a salivary serous component recovery instrument; 7, a recovery hole; 8, a saliva absorber compressing portion; 9, a saliva absorber insertion port/finger-engaging member; 10, a saliva secretion component-containing bactericidal mouthwash; 11, a bactericidal component and/or saliva secretion promoting component-containing sublingual surplus water absorber; and 12, a salivary serous component.

### DESCRIPTION OF THE PREFERRED EMBODIMENTS

The bactericidal component and antibacterial component used in the present invention are not limited, as long as they can be used in the oral cavity without affecting the human body, they sterilize oral bacteria which degrade glucose, and they do not impair saliva tests including glucose determination. Examples of the bactericidal component are quaternary ammonium salts such as cetylpyridinium chloride, benzalkonium chloride, and benzethonium chloride. As the antibacterial component, chitin/chitosan is named, for example. Among these bactericidal and antibacterial components, a single component or a plurality of components can be used.

The saliva secretion promoting component is not limited, if it is a food additive which does not impair saliva tests including glucose determination. As a sweetening agent, for example, substances other than glucose, such as aspartame·L-phenylalanine compounds, xylitol and sorbitol, are named. Examples of a sour agent are citric acid, gluconic acid, acetic acid, lactic acid, and malic acid. Examples of a flavor are 1-menthol and components with fruit aroma. Among these additives, a single component or a plurality of components can be used.

The water-soluble polymer is not limited, as long as it is a food additive which does not impair saliva tests including glucose determination. For example, carboxymethylcellulose and microparticle silicon dioxide can be used as a single component or a plurality of components.

The following is a description of the swab type saliva sampling instrument:

The material for the saliva absorber is not limited, if it is usable in the oral cavity without exerting influence on the human body, if it can contain the bactericidal component, the antibacterial component, and the saliva secretion promoting component, if it is free from nonspecific unintended adsorption to sampled saliva or contamination of saliva, if it is formed from a material absorbing saliva, and if it has such mechanical strength as not to be damaged during use. For example, cotton, cellulose, polyurethane, polyester, rayon, nonwoven fabric, or a fiber blend of these can be used.

The materials for the piston rod, the gasket, and the pressing pad are not limited, if they do not influence the human body, if they can be used in the oral cavity, if they can incorporate the bactericidal component, the antibacterial component, and the saliva secretion promoting component and can be coated with these components, if they are free from nonspecific unintended adsorption to sampled oral liquid secreta or contamination of oral liquid secreta, if they have flexibility, water resistance and satisfactory processability, and if they have such mechanical strength as not to be damaged during use. Examples of such materials are paper, silicone, nylon, polyester, polyethylene, polyethylene terephthalate, polystyrene, and polypropylene. The piston rod may be a paper shaft, the gasket may be a silicone tube, the pressing pad may be made of polypropylene. By making them of paper, they can be produced inexpensively, and their satisfactory adhesion to the saliva absorber and their satisfactory processability make their use possible.

The material for the salivary serous component recovery instrument is not limited, if it does not influence the human body, if it can incorporate and can be coated with the bactericidal component and the antibacterial component, if it is free from nonspecific unintended adsorption to sampled oral liquid secretions, if it has flexibility, water resistance and satisfactory processability, and if it has such mechanical strength as not to be damaged during use. Examples of such materials are silicone, nylon, polyester, polyethylene, polyethylene terephthalate, polystyrene, and polypropylene.

The recovery hole located at the tip of the salivary serous component recovery instrument is not limited, if it communicates with the saliva absorber compressing portion, if a trace-amount piece of the same component as that of the saliva absorber is present as a filter layer halfway through the hole, if the recovery hole achieves a structure and a function which permit the salivary serous component to be recovered through the recovery hole when the saliva sampling instrument is pressed toward the recovery hole, and if the recovery hole has a shape which enables a recovered sample to be spotted with good efficiency.

The saliva absorber insertion port/finger-engaging member is not limited, if it has a shape which can easily guide the insertion of the saliva absorber, and if it has such a shape as to permit engagement with fingers and has such mechanical strength as not to undergo damage or deformation, when the saliva sampling instrument is pressed to compress the saliva absorber.

No limitations are imposed on the degradation of the saliva-intrinsic inhibitor, if the function of completing degradation when recovering the salivary serous component is achieved by immobilizing a degrading substance to, or incorporating the degrading substance into, the inner wall surface and/or the filter layer of the salivary serous component recovery instrument. For example, ascorbic acid oxidase is named as a degrading substance for ascorbic acid, and uricase is named as a degrading substance for uric acid. Various degrading substances can be used according to test items on saliva. If the salivary test item is uric acid, uricase cannot be used.

### Examples

The present invention will now be described concretely with reference to Examples, but the present invention is not limited to these Examples.

### Example 1 Saliva sampling instrument and salivary serous component recovery instrument

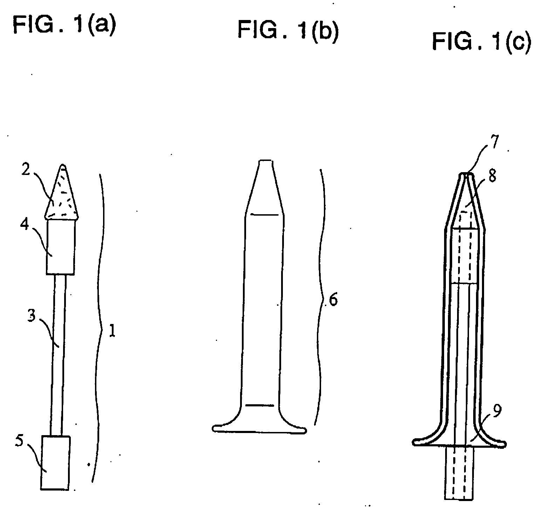

An example of a saliva sampling instrument and an example of a salivary serous component recovery instrument according to the present example are shown in FIGS. 1(a) to 1(c).

A swab type saliva sampling instrument 1 was composed of a saliva absorber 2 comprising cotton having chitin/chitosan as an antibacterial component on its surface, a piston rod 3 as a paper shaft, and a gasket 4 and a pressing pad 5 formed on the piston rod 3, each of the gasket 4 and the pressing pad 5 likewise comprising paper. The saliva absorber was formed in a conical shape with a volume of 65 mm³.

A syringe type salivary serous component recovery instrument 6 was molded from polyethylene. A compressing portion 8 of the instrument 6 was formed in a conical shape with an internal volume of 20 mm³, and 1 mm³ of the above-mentioned cotton was disposed halfway between the compressing portion 8 and a recovery hole 7 of the instrument 6 to serve as a filter. The diameter of the recovery hole was set at 1 mm or less to prevent dropout of the cotton during recovery of a salivary serous component. A saliva absorber insertion port/finger-engaging member 9 had a bell-bottom shape with a radius of 5 mm.

### Example 2 Saliva sampling and recovery kit

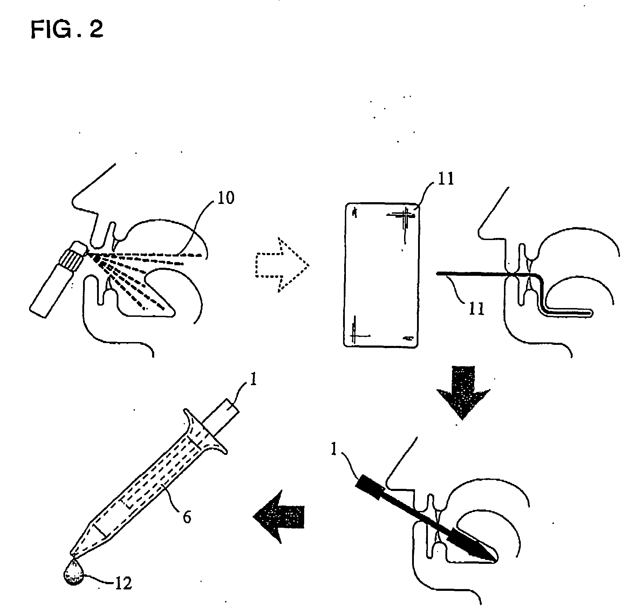

An example of a saliva sampling and recovery kit and an example of a method of using the kit according to the present example are shown in FIG. 2.

Cetylpyridinium chloride (0.05 wt.%; Takeda Chemical Industries) as a bactericidal component, and a sweetener, a sour agent, and a flavor (POLA FOODS) as saliva secretion promoting components were compounded to prepare a solution reproducing sour-sweetness, which was used as a saliva secretion component-containing bactericidal mouthwash 10.

A solution further containing carboxymethylcellulose (Dai-ichi Kogyo Seiyaku) in addition to the above compounded solution was sprayed over a nonwoven fabric (80 g/m²) (OMIKENSHI) containing chitin/chitosan as an antibacterial component, followed by drying, to form a sublingual surplus water absorber 11.

The above compounded solution was sprayed over the saliva absorber 2, followed by drying, to form the saliva sampling instrument 1.

A kit comprising the three articles, i.e., the saliva secretion component-containing bactericidal mouthwash 10, the sublingual surplus water absorber 11, and the saliva sampling instrument 1/salivary serous component recovery instrument 6, was used in the following manner: The saliva secretion component-containing bactericidal mouthwash 10 was sprayed into the mouth, particularly over a sublingual region. Washing of the mouth is not limited, but may be, for example, gargling or dropping to the mouth. Then, the sublingual surplus water absorber 11 was placed between the sublingual region and the bottom of the oral cavity. As a result, the incorporated components were dissolved again to present a taste, and water in the sublingual region was absorbed, whereby the sublingual region was dewatered.

After the above-described pretreatment, the saliva sampling instrument 1 was immediately placed between the sublingual region and the bottom of the oral cavity. Satisfactory saliva secretion by taste and odor was confirmed. Then, the swollen saliva absorber 2 was compressed by the salivary serous component recovery instrument 6, whereby serous component saliva 12 deprived of a viscous substance, apparently mucin, was obtained through the recovery hole 7.

In subjects who did not show diseases such as xerostomia, the saliva sampling instrument 1 was placed in the mouth for 30 seconds, and then compressed. Serous component saliva 12 was obtained in an amount of 25 µl.

To investigate the degradation of a saliva-intrinsic inhibitor, a solution of ascorbic acid oxidase (TOYOBO) was sprayed over the inner wall surface of the salivary serous component recovery instrument 6 and the filter disposed on the inner surface inwardly of the recovery hole 7, followed by drying, with ascorbic acid being targeted. Then, the saliva absorber 2 was wetted with saliva incorporating ascorbic acid, and then compressed by the salivary serous component recovery instrument 6 to recover serous component saliva 12. Degradation of ascorbic acid by redissolved ascorbic acid oxidase was observed. Similarly, when uricase (TOYOBO) was applied, with uric acid being targeted, degradation of uric acid was noted.

In addition to the above-mentioned kit constituted of the three articles, the present invention provides a kit composed of two articles. i.e., the sublingual surplus water absorber 11 and the saliva sampling instrument 1/salivary serous component recovery instrument 6, or a kit consisting only of the saliva sampling instrument 1/salivary serous component recovery instrument 6, except the pretreatment articles.

According to the above Examples, particular shapes are illustrated in connection with the simple instrument for sampling and recovery of saliva. However, the present invention is not limited to these shapes.

Nor is the present invention limited to the above Examples in regard to the saliva secretion component-containing bactericidal mouthwash 10 and the sublingual surplus water absorber 11 that are used concomitantly with the saliva sampling and recovery instrument.

## Claims

1. An instrument for sampling of saliva and recovery of a salivary serous component, arranged to sample saliva secreted mainly from sublingual gland and submandibular gland, and remove a viscous component in saliva, thereby recovering serous saliva, and
wherein the instrument comprises a swab type sampling instrument and a syringe type recovery instrument,
the swab type sampling instrument has, at one end thereof, a flexible saliva absorber having a shape of a cone or a semisphere and containing at least a bactericidal component and/or an antibacterial component, or both the bactericidal component and/or the antibacterial component and a saliva secretion promoting component,
the swab type sampling instrument has, at a bottom portion of the cone or semisphere, a gasket having a diameter conforming to an internal diameter of the syringe type recovery instrument,
the syringe type recovery instrument has, at one end thereof, a wide open receiving port for facilitating insertion of the saliva absorber,
the syringe type recovery instrument has, at an opposite end thereof, a recovery port for saliva having a filtering function, and a conical or semispherical absorber compressing portion having a slightly smaller internal volume than a volume of the saliva absorber, and
the syringe type recovery instrument has, on an inner surface thereof, a substance for degrading a saliva-intrinsic inhibitor.

2. The instrument for sampling of saliva and recovery of a salivary serous component according to claim 1, wherein
the bactericidal component and the antibacterial component at least have functions of killing, or suppressing growth of, oral bacteria which degrade glucose,
the bactericidal component and the antibacterial component comprise a single component or a plurality of components,
the bactericidal component is cetylpyridinium chloride, benzalkonium chloride, or benzethonium chloride, and
the antibacterial component is chitin/chitosan.

3. The instrument for sampling of saliva and recovery of a salivary serous component according to claim 1 or 2,
wherein
the saliva secretion promoting component is at least three of a sweetener, a sour agent, and a flavor which do not impair assay of saliva recovered.

4. The instrument for sampling of saliva and recovery of a salivary serous component according to any one of claims 1 to 3, wherein
the saliva absorber comprises a single component or a plurality of components selected from cotton, cellulose, nylon, polyurethane, polyester, and rayon, and has a function of absorbing saliva promptly, and further has a function of catching a viscous component in saliva among fibers of the absorber.

5. The instrument for sampling of saliva and recovery of a salivary serous component according to any one of claims 1 to 4, wherein
the recovery hole at least comprises a single hole or a plurality of holes, and has the filtering function because the saliva absorber comprising a single component or a plurality of components is disposed in a trace amount halfway through a hole progressively thinning toward an exit of the recovery hole.

6. The instrument for sampling of saliva and recovery of a salivary serous component according to any one of claims 1 to 5, wherein
the substance which degrades the saliva-intrinsic inhibitor is immobilized to and incorporated into an inner wall surface of the syringe type recovery instrument and/or the filter, and has a function of specifically degrading a salivary-intrinsic substance which inhibits assay when the salivary serous component is recovered.

7. The instrument for sampling of saliva and recovery of a salivary serous component according to any one of claims 1 to 6, wherein
the volume of the saliva absorber is 150 mm³ or less.

8. A kit for sampling of saliva and recovery of a salivary serous component, comprising:
the instrument for sampling of saliva and recovery of a salivary serous component according to any one of claims 1 to 7;
a mouthwash containing at least the bactericidal component and the saliva secretion promoting component; and
a sublingual surplus water absorber being a non-woven fabric containing at least the saliva secretion promoting component and a water-soluble polymer.

9. A kit for sampling of saliva and recovery of a salivary serous component, comprising:
a sublingual surplus water absorber being a non-woven fabric containing at least the bactericidal component, the saliva secretion promoting component, and a water-soluble polymer; and
the instrument for sampling of saliva and recovery of a salivary serous component according to any one of claims 1 to 7.

10. The kit for sampling of saliva and recovery of a salivary serous component according to claim 8 or 9,
wherein
the water-soluble polymer has functions of improving resolubility of substances incorporated into the surplus water absorber and imparting moderate strength to the flexible non-woven fabric.

11. A method for sampling saliva and recovering a salivary serous component, while performing sterilization and promotion of saliva secretion, comprising:
performing a three-stage operation using the instrument or kit according to any one of claims 1 to 10, and comprising mouth washing by the mouthwash, sublingual dewatering by the surplus water absorber, and sampling of saliva and recovery of the salivary serous component; or
performing a two-stage operation using the instrument or kit according to any one of claims 1 to 10, and comprising sublingual dewatering by the surplus water absorber, and sampling of saliva and recovery of the salivary serous component; or
performing a one-stage operation using the instrument or kit according to any one of claims 1 to 10, and comprising sampling of saliva and recovery of the salivary serous component.
